# EUROPÄISCHE PATENTANMELDUNG

(11) **EP 3 293 194 A1**
(43) Veröffentlichungstag der Anmeldung: **14.03.2018**
(21) Anmeldenummer: 16187587.7
(22) Anmeldetag: 07.09.2016
(51) Int. Cl.: C07F 15/00, B01J 31/16, C07B 41/06, C07C 45/50, C07F 9/6574

(54) **PHOSPHITE MIT EINEM SILYLOXYPHENOL UND EINEM BIPHENOL**

(71) Anmelder: Evonik Degussa GmbH, 45128 Essen (DE)
(72) Erfinder: DYBALLA, Katrin Marie, 45657 Recklinghausen (DE); FRANKE, Robert, 45772 Marl (DE)

(57) **Zusammenfassung**

Beansprucht werden Phosphite mit einem Silyloxyphenol und einem Biphenol gemäss Markush Formel (I), Komplexe damit sowie die Verwendung als Liganden in Komplexen zur Hydroformylierung. 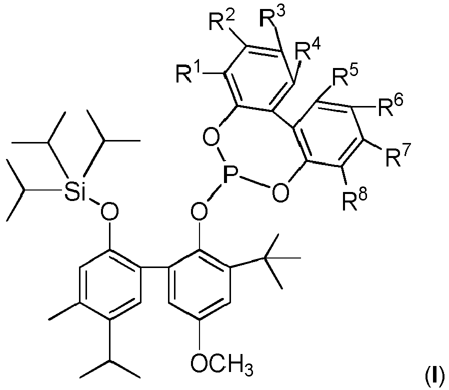 Eine besondere Ausführungsform ist die Verbindung (2):

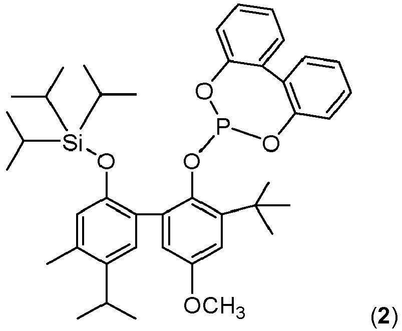

## Beschreibung

Die Erfindung betrifft Phosphite mit einem Silyloxyphenol und einem Biphenol, sowie deren Verwendung als Liganden in der Hydroformylierung.

Phosphorhaltige Verbindungen spielen als Liganden in einer Vielzahl von Reaktionen eine entscheidende Rolle. Hierzu zählen Phosphitliganden, also Verbindungen, die P-O-Bindungen enthalten, die in der Hydrierung, Hydrocyanierung und vor allem in der Hydroformylierung Anwendung finden.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Der Erfindung lag die Aufgabe zugrunde, neue Verbindungen bereitzustellen, welche in einem Verfahren zur Hydroformylierung von Olefinen eingesetzt werden können. Des Weiteren soll ein Verfahren zur Hydroformylierung von Olefinen bereitgestellt werden, welches zu einer guten Ausbeute an Aldehyd führt.

Gelöst wird die Aufgabe durch eine Verbindung gemäß Anspruch 1, beziehungsweise durch ein Verfahren gemäß Anspruch 11.

Verbindung gemäß der allgemeinen Formel (**I**): 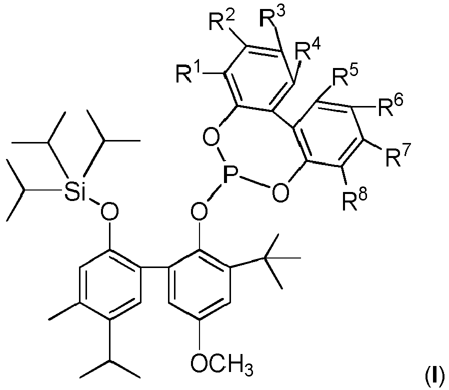 wobei die Reste R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl.

Der Ausdruck -(C₆-C₂₀)-Aryl umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Die Erläuterungen zum Ausdruck -(C₆-C₂₀)-Aryl gelten auch für die Alkylgruppen in -O-(C₆-C₂₀)-Aryl.

Unter Halogen werden Cl, F, Br, I zusammengefasst. Hierbei ist Cl bevorzugt.

In einer Ausführungsform sind die Reste R¹, R², R³, R⁴, R⁵, R⁶, R⁷, R⁸ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl.

In einer Ausführungsform sind die Reste R¹, R², R³, R⁴, R⁵, R⁶, R⁷, R⁸ jeweils unabhängig voneinander ausgewählt aus:
-H, -(C₁-C₁₂)-Alkyl, -(C₆-C₂₀)-Aryl.

In einer Ausführungsform sind die Reste R¹, R², R³, R⁴, R⁵, R⁶, R⁷, R⁸ jeweils unabhängig voneinander ausgewählt aus:
-H, -(C₁-C₁₂)-Alkyl, -(C₆-C₂₀)-Aryl, Halogen.

In einer Ausführungsform sind die Reste R¹, R², R³, R⁴, R⁵, R⁶, R⁷, R⁸ jeweils unabhängig voneinander ausgewählt aus:
-H, -(C₁-C₁₂)-Alkyl.

In einer Ausführungsform sind die Reste R¹, R², R³, R⁴, R⁵, R⁶, R⁷, R⁸ jeweils unabhängig voneinander ausgewählt aus:
-H, -(C₆-C₂₀)-Aryl.

In einer Ausführungsform stehen die Reste R¹, R³, R⁶, R⁸ für -H.

In einer Ausführungsform stehen die Reste R², R⁴, R⁵, R⁷ für -H.

In einer Ausführungsform weist die Verbindung die folgenden Formel (2) auf:

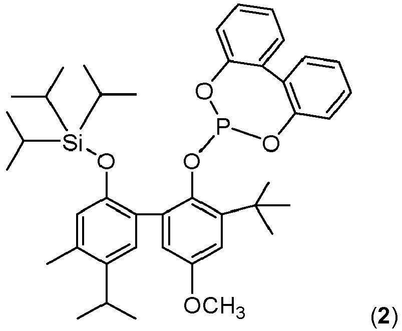

Neben der Verbindung wird auch ein Komplex beansprucht, welcher die Verbindung umfasst.

Komplex umfassend:
- eine zuvor beschriebene Verbindung,
- ein Metallatom ausgewählt aus: Rh, Ru, Co, Ir.

Hierbei ist Rh bevorzugt.

Des Weiteren wird die Verwendung einer zuvor beschriebenen Verbindung als Ligand in einem Ligand-Metall-Komplex zur Katalyse einer Hydroformylierungsreaktion beansprucht.

Verwendung einer zuvor beschriebenen Verbindung als Ligand in einem Ligand-Metall-Komplex zur Katalyse einer Hydroformylierungsreaktion.

Neben den Verbindungen an sich wird auch ein Verfahren zur Hydroformylierung von Olefinen beansprucht, in welchem diese Verbindungen zum Einsatz kommen.

Verfahren umfassend die Verfahrensschritte:
a) Vorlegen eines Olefins,
b) Zugabe eines zuvor beschriebenen Komplexes,
oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer Variante des Verfahrens ist das Metallatom Rh.

In einer Variante des Verfahrens umfasst die Substanz (= Komplexvorstufe) Cyclooctadien.

In einer Variante des Verfahrens handelt es sich bei der Substanz (= Komplexvorstufe) um [(acac)Rh(COD)].

In einer Variante des Verfahrens wird das Reaktionsgemisch im Verfahrensschritt d) auf eine Temperatur im Bereich von 100 ° C bis 140 °C erwärmt.

Im Folgenden wird die Erfindung anhand von Ausführungsbeispielen näher erläutert.

### Experimentelles

Alle präparativen Arbeiten erfolgten unter Anwendung der Schlenk-Technik unter Argon als Schutzgas. Trockenes und luftfreiese Toluol und Tetrahydrofuran wurden mittels eines Pure Solv. MD-7 Systems erhalten und unter Argon aufbewahrt. Triethylamin wurde vor dem Einsatz unter Argon von Natriumketyl destilliert. Phosphortrichlorid (Aldrich) wurde vor dem Einsatz unter Argon destilliert. Alle präparativen Arbeiten erfolgten in ausgeheizten Gefäßen. Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Synthesen

### Vorstufe:

Die Vorstufe wurde nach einem bekannten Verfahren synthetisiert:

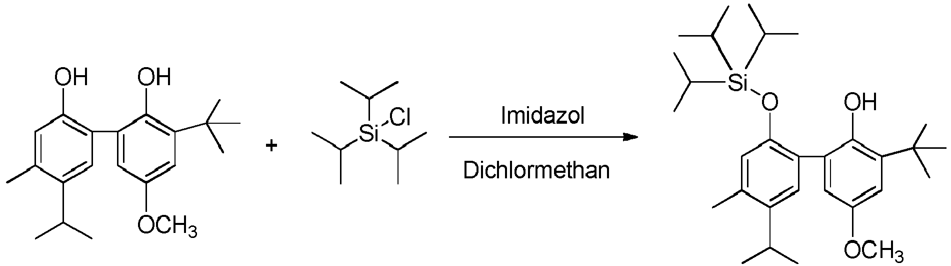

### a) 2-((3-(tert-Butyl)-5'-isopropyl-5-methoxy-4'-methyl-2'-((triisopropylsilyl)oxy)-[1,1'-biphenyl]-2-yl)oxy)-4,4,5,5-tetraphenyl-1,3,2-dioxaphospholan (Vergleichsligand 1)

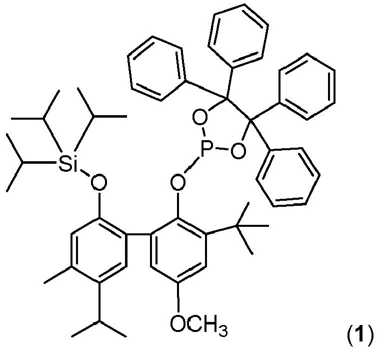

Zu einer bei -20 °C gerührten Lösung des Silyloxyphenols (0,813 g, 1,676 mmol) in THF (7 ml) wird tropfenweise eine 0,53 molare Lösung von n-Butyllithium in Hexan gegeben (3,16 ml; 1,675 mmol). Man rührt noch 20 min, lässt dann auf Raumtemperatur erwärmen und tropft die erhaltene Lösung bei 0 °C zu einer Lösung von 2-Chlor-4,4,5,5-tetraphenyl-1,3,2-dioxaphospholan (0,722 g; 1,676 mmol) in THF (7 ml). Man lässt noch 30 min rühren, erwärmt dann und rührt über Nacht bei Raumtemperatur. Es wird filtriert, anschließend das Lösungsmittel im Vakuum entfernt und der Rückstand bei Raumtemperatur und 0,1 mbar getrocknet. Der erhaltene Feststoff wird in Toluol (8 ml) gelöst. Man filtriert und engt das Filtrat im Vakuum bis zur Trockne ein. Das Rohprodukt wird mit Heptan (3 ml) verrührt, man filtriert, wäscht mit Heptan (1 ml) und trocknet den weißen Feststoff im Vakuum. Ausbeute: 1,056 g (1,201 mmol; 72%).

Elementaranalyse (ber. für C₅₆H₆₇O₅PSi = 879,15 g/Mol): C 76,65 (76,50); H 7,88 (7,68); P 3,52 (3,50)%.

³¹P-NMR (CD₂Cl₂): 147,5 (s) ppm.
¹H-NMR (CD₂Cl₂): 0,95-1,27 (m, 33H); 2,45 (s, 3H), 3,12 (Septett, 1H); 3,78 (s, 3H); 6,65 (d, *J*_{HH} = 3,0 Hz; 1 H); 6,83-7,43 (m, 23 H) ppm.
¹³C-NMR (CD₂Cl₂) δ = 13.3, 17.9, 18.3, 18.3, 19.6, 23.2, 23.9, 29.3, 30.4, 35.1, 55.8, 94.7 (d, *J*_{CP} = 8,7 Hz), 95.3 (d, *J*_{CP} = 9,0 Hz), 113.7, 121.1, 126.8, 127.0, 127.1, 127.2, 127.3, 127.4, 127.7, 128.3, 128.5, 128.7, 128.7, 129.0, 129.2, 129.5, 129.6, 130.0, 131.3, 134.6, 136.3, 138.9, 142.4, 142.6, 143.0, 143.5, 143.6, 143.7, 143.8, 152.2, 155.0 ppm

ESI-TOF HRMS: m/e 901,43907 (M+Na)⁺.

### b) 6-((3-(tert-Butyl)-5'-isopropyl-5-methoxy-4'-methyl-2'-((triisopropylsilyl)oxy)-[1,1'-biphenyl]-2-yl)oxy)dibenzo[d,f][1,3,2]dioxaphosphepin (Ligand 2)

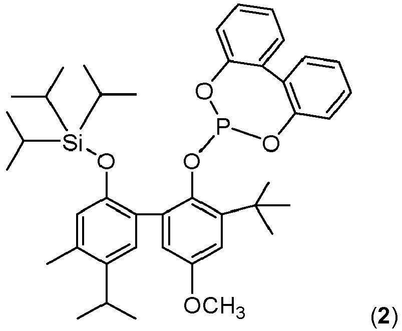

Zu einer bei -20 °C gerührten Lösung des Silyloxyphenols (0,684 g, 1,411 mmol) in THF (8 ml) wird tropfenweise eine 0,32 molare Lösung von n-Butyllithium in Hexan gegeben (4,4 ml; 1,411 mmol). Man rührt noch 20 min, lässt dann auf Raumtemperatur erwärmen und tropft die erhaltene Lösung bei 0 °C zu einer Lösung von 6-Chlordibenzo[*d*,*f*][1,3,2]dioxaphosphepin (0,371 g; 1,482 mmol) in THF (4 ml). Man lässt noch 30 min rühren, erwärmt dann und rührt über Nacht bei Raumtemperatur. Es wird filtriert, anschließend das Lösungsmittel im Vakuum entfernt und der Rückstand bei Raumtemperatur und 0,1 mbar getrocknet. Der erhaltene Feststoff wird in Toluol (12 ml) gelöst. Man filtriert und engt das Filtrat im Vakuum bis zu einem viskosen Feststoff ein. Dieser wird in 10 ml Heptan aufgenommen, die Lösung wird filtriert und zur Trockne eingeengt. Diese Prozedur wird wiederholt. Es werden 0,261 g (0,373 mmol; 26%) an analysenreinem Produkt erhalten.

Elementaranalyse (ber. für C₄₂H₅₅O₅PSi = 698,95 g/Mol): C 72,10 (72,17); H 8,05 (7,93); P 4,33 (4,43)%.

³¹P-NMR (CD₂Cl₂): 147,6 (s) ppm.
¹H-NMR (CD₂Cl₂): 0,95 (d, *J*_{HH}= 7,3Hz; 18H); 1,13-1,29 (m, 9H); 2,42 (s, 3H), 3,18 (Septett, *J*_{HH}= 6,8 Hz; 1 H); 3,80 (s, 3H); 6,64 (d, *J*_{HH} = 3,2 Hz; 1 H); 6,74 (s, br, 1 H), 6,92-6,99 (m, 3H); 7,23 (s, br, 1 H), 7,25-7,38 (m, 4H); 7,46-7,49 (m, 2H) ppm.
¹³C-NMR (CD₂Cl₂) δ = 13.2, 18.1, 19.5, 23.5, 23.7, 29.2, 30.7, 35.7, 55.9, 114.0, 114.2, 120.2, 122.9, 125.2, 128.1, 129.1, 129.8, 130.1, 131.5, 133.6, 136.6, 138.7, 142.7, 143.8, 149.8, 152.5, 155.1 ppm.

ESI-TOF HRMS: m/e 699,36295 (M+H)⁺.

### Durchführung der Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl behandelt und unter Argon destilliert. Das eingesetzte Olefin, n-Octene (Oxeno GmbH, Octenisomerengemisch aus 1-Octen: 3,3 %; *cis*+*trans*-2-Octen: 48,5%; *cis*+*trans*-3-Octen: 29,2%; *cis*+*trans*-Octen-4: 16,4 %; gerüstisomere Octene: 2,6 %), wurden mehrere Stunden über Natrium am Rückfluß erhitzt und unter Argon destilliert.
Für die Versuche wurden im Autoklaven unter Argonatmosphäre Lösungen der Katalysatorvorstufe und des Liganden gemischt. Als Komplexvorstufe (= Katalysatorvorstufe) kam [(acac)Rh(COD)] (Umicore, acac = Acetylacetonat-Anion; COD = 1,5-Cyclooctadien) zum Einsatz. Für Versuche mit einer Konzentration von 100 ppm-m Rhodium wurden 10 ml einer 4,31 millimolaren Lösung in den Autoklaven gegeben. Anschließend wurde die entsprechende Masse des Liganden in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurde als Olefin eingefüllt: n-Octene (10,70 g; 95,35 mmol). Der Autoklav wurde bei einem Gesamtgasdruck (Synthesegas: Linde; H₂ (99,999%) : CO (99,997%) = 1:1) von 12 bar für den Enddruck von 20 bar unter Rühren (1500 U/min) aufgeheizt. Nach Erreichen der Reaktionstemperatur von 120 °C wurde der Synthesegasdruck auf 19,5 bar für einen Enddruck von 20 bar erhöht und das Olefin(-gemisch) mit einem in der Druckpipette eingestellten Überdruck von ca. 3 bar in den Autoklaven gepresst. Die Reaktion wurde bei konstantem Druck von 20 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm.

Die Ergebnisse der Hydroformylierungsversuche sind in der nachfolgenden Tabelle zusammengestellt:

**Tabelle 1:**

| **Substrat: n-Octene, 20 bar CO/H₂** | | | | |
|---|---|---|---|---|
| **Ligand** | **p [bar]** | **T [°C]** | **L/Rh** | **Ausbeute Aldehyd (%)** |
| **1** | 20 | 120 | 5 | 74 |
| **2*** | 20 | 120 | 5 | 99 |

| | | | | |
|---|---|---|---|---|
| * erfindungsgemäße Verbindung bzw. erfindungsgemäßes Verfahren Reaktionsbedingungen: *t* = 4h, [Rh] = 100 ppm-m, Lösungsmittel: Toluol | | | | |

Wie die Versuchsergebnisse zeigen, wird die Aufgabe durch das erfindungsgemäße Verfahren gelöst.

## Patentansprüche

1. Verbindung gemäß der allgemeinen Formel (I): 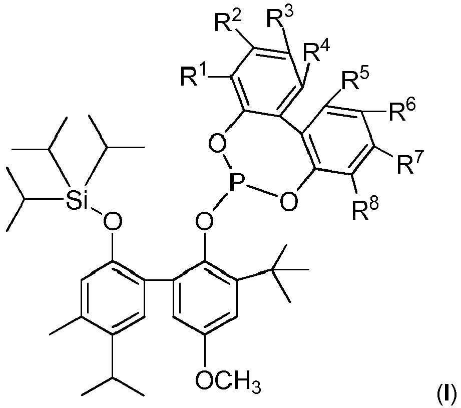 wobei die Reste R¹, R², R³, R⁴, R⁵, R⁶, R⁷, R⁸ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl, -Halogen.

2. Verbindung nach Anspruch 1,
wobei die Reste R¹, R², R³, R⁴, R⁵, R⁶, R⁷, R⁸ jeweils unabhängig voneinander ausgewählt sind aus:
- H, -(C₁-C₁₂)-Alkyl, -O-(C₁-C₁₂)-Alkyl, -(C₆-C₂₀)-Aryl, -O-(C₆-C₂₀)-Aryl.

3. Verbindung nach einem der Ansprüche 1 oder 2,
wobei die Reste R¹, R², R³, R⁴, R⁵, R⁶, R⁷, R⁸ jeweils unabhängig voneinander ausgewählt sind aus:
- H, -(C₁-C₁₂)-Alkyl, -(C₆-C₂₀)-Aryl.

4. Verbindung nach einem der Ansprüche 1 bis 3,
wobei die Reste R¹, R², R³, R⁴, R⁵, R⁶, R⁷, R⁸ jeweils unabhängig voneinander ausgewählt sind aus:
- H, -(C₁-C₁₂)-Alkyl.

5. Verbindung nach einem der Ansprüche 1 bis 3,
wobei die Reste R¹, R², R³, R⁴, R⁵, R⁶, R⁷, R⁸ jeweils unabhängig voneinander ausgewählt sind aus:
- H, -(C₆-C₂₀)-Aryl.

6. Verbindung nach einem der Ansprüche 1 bis 5,
wobei die Reste R¹, R³, R⁶, R⁸ für -H stehen.

7. Verbindung nach einem der Ansprüche 1 bis 6,
wobei die Reste R², R⁴, R⁵, R⁷ für -H stehen.

8. Verbindung nach einem der Ansprüche 1 bis 7,
gemäß der folgenden Formel (2):

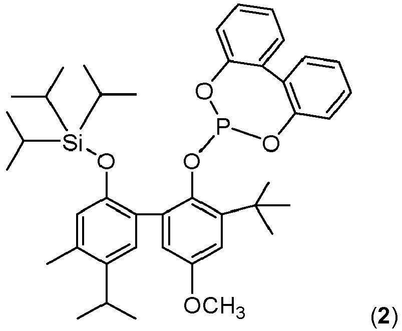

9. Komplex umfassend:
- eine Verbindung nach einem der Ansprüche 1 bis 8,
- ein Metallatom ausgewählt aus: Rh, Ru, Co, Ir.

10. Verwendung einer Verbindung nach einem der Ansprüche 1 bis 8,
als Ligand in einem Ligand-Metall-Komplex zur Katalyse einer Hydroformylierungsreaktion.

11. Verfahren umfassend die Verfahrensschritte:
a) Vorlegen eines Olefins,
b) Zugabe eines Komplexes nach Anspruch 9,
oder einer Verbindung nach einem der Ansprüche 1 bis 8 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

12. Verfahren nach Anspruch 11,
wobei das Metallatom Rh ist.
